# EUROPÄISCHE PATENTSCHRIFT

(11) **EP 1 828 081 B1**
(45) Veröffentlichungstag und Bekanntmachung des Hinweises auf die Patenterteilung: **29.08.2018**
(21) Anmeldenummer: 05806349.6
(22) Anmeldetag: 11.10.2005
(51) Int. Cl.: C07C 7/04, C07C 7/08

(54) **VERFAHREN ZUM ENTFERNEN VON SAUERSTOFFHALTIGEN ORGANISCHEN VERBINDUNGEN AUS GEMISCHEN VERSCHIEDENER KOHLENWASSERSTOFF-VERBINDUNGEN**
DEVICE FOR REMOVING OXYGEN-CONTAINING ORGANIC COMPOUNDS FROM MIXTURES OF VARIOUS HYDROCARBON COMPOUNDS
PROCÉDÉ POUR ÉLIMINER DES COMPOSÉS ORGANIQUES CONTENANT DE L'OXYGÈNE DE MÉLANGES DE DIFFÉRENTS COMPOSÉS D'HYDROCARBURES

(30) Priorität: 29.10.2004 DE 102004052658
(43) Veröffentlichungstag der Anmeldung: 05.09.2007
(73) Patentinhaber: Air Liquide Global E&C Solutions Germany GmbH, 60439 Frankfurt am Main (DE)
(72) Erfinder: JENSEN, Sandra, 60439 Frankfurt am Main (DE); ROTHAEMEL, Martin, 60437 Frankfurt am Main (DE); KOEMPEL, Harald, 63263 Neu-Isenburg (DE); BACH, Hermann, 56412 Heiligenroth (DE); BIRKE, Gerhard, 60839 Frankfurt am Main (DE)
(86) Internationale Anmeldenummer: PCT/EP2005/010921
(87) Internationale Veröffentlichungsnummer: WO 2006/048098

(56) Entgegenhaltungen:
- WO-A-03/020671
- WO-A-03/020678

## Beschreibung

Die Erfindung betrifft ein Verfahren zum Entfernen von sauerstoffhaltigen organischen Verbindungen (Oxygenaten) aus Gemischen verschiedener Kohlenwasserstoff-Verbindungen unter gleichzeitiger Trennung der Kohlenwasserstoffverbindungen in Einzelfraktionen.

Kohlenwasserstoff-Verbindungen sind Grundelemente der chemischen Industrie und Ausgangsstoff für eine Vielzahl von Produkten. Industriell verwertbare Kohlenwasserstoff-Verbindungen können durch die Umwandlung von fossilen festen, flüssigen oder gasförmigen Brennstoffen erzeugt werden. Ein Beispiel hierfür ist die verstärkte Nutzung von Erdgas für die Herstellung von flüssigen Brennstoffen und Chemikalien. So kann Erdgas zu Synthesegas umgesetzt werden und Synthesegas für die Erzeugung von Wasserstoff, beispielsweise für den Einsatz in Raffinerien und Brennstoffzellen, für die Erzeugung von Fischer-Tropsch-Produkten, wie schwefelfreie Kraftstoffe, Schmiermittel, Wachse und α-Olefine, für die Erzeugung von DME, zum Beispiel für den Einsatz in Gasturbinen und Brennstoffzellen und ganz besonders für die Erzeugung von Methanol als Ausgangsstoff für die Gewinnung von Formaldehyd, Lösungsmittel, Methyltertiärbutylether, synthetischen Kraftstoffen, Essigsäure, Olefine, etc., verwendet werden.

Typischerweise fallen die Kohlenwasserstoffverbindungen bei den primären Erzeugungsprozessen in Form von Gemischen an, die mittels Trennverfahren - vor allem fraktionierter Destillation - in Einzelfraktionen oder Reinstoffe getrennt werden müssen. Die bei den herkömmlichen Verfahren verwendete Verschaltung der Trennapparate führt zu großen Abmessungen der einzelnen Ausrüstungsteile sowie einem hohen spezifischen Verbrauch an Betriebsmitteln. Demnach kommt der optimalen Gestaltung des Trennprozesses eine große Bedeutung zu. Die Kohlenwasserstoff-Verbindungen sollen dabei in möglichst reiner Form ohne Anwesenheit von sauerstoffhaltigen organischen Verbindungen (Oxygenaten) erzeugt werden. Als Oxygenate werden Verbindungen mit mindestens einem Kohlenwasserstoffgerüst und geringem Sauerstoffanteil bezeichnet.
Nach dem Stand der Technik wurden die Oxygenate durch eine klassisch verschaltete Destillation oder eine physikalische Wäsche von den Kohlenwasserstoff-Verbindungen getrennt. Dies ist aber insbesondere bei großtechnischen Anlagen sehr aufwendig und teuer. In den Schriften WO 03/020671, WO 03/020672 und WO 03/020678 werden Verfahren zur Extraktivdestillation von Olefinen beschrieben.
Es ist die Aufgabe der vorliegenden Erfindung, ein Verfahren zum Entfernen von sauerstoffhaltigen organischen Verbindungen (Oxygenaten) aus Gemischen verschiedener Kohlenwasserstoff-Verbindungen mit einer Kohlenstoffzahl (C-Zahl) von C-1 bis C-9 bereitzustellen, bei dem der verbleibende Anteil der Oxygenate im Hauptwertproduktstrom, z.B. einem Olefinstrom auf unter 1 ppm reduziert wird, sowie eine Trennung in Teilfraktionen erreicht wird, bei gleichzeitiger Minimierung der Apparateabmessungen und der spezifischen Betriebsm ittelverbräuche.
Die Lösung dieser Aufgabe erfolgt dadurch, dass das Gemisch aus Kohlenwasserstoffen und sauerstoffhaltigen organischen Verbindungen in einem zweistufigen gemäß Anspruch 1 verarbeitet wird. Typischerweise liegen derartige Kohlenwasserstoffströme - z.B. nach einer Kompression - in einer 2-phasigen Form vor, wobei sich die schwereren Kohlenwasserstoffe in der flüssigen Phase befinden. Bei der vorliegenden Erfindung werden diese beiden Phasen nicht wie herkömmlich gemeinsam in eine Destillationskolonne aufgegeben, sondern separat in zwei Destillationskolonnen. Aus der Flüssigphase wird in der ersten Kolonne eine leichte Fraktion abgetrennt, in der sich das Hauptwertprodukt und Oxygenate befinden.
Die Gasphase wird zusammen mit der leichten Fraktion der ersten Kolonne in die zweite Kolonne aufgegeben. Bei dieser zweiten Kolonne handelt es sich um eine Extraktivdestillationskolonne. Das Gemisch wird in einen leichten und einen schweren Kohlenwasserstoffschnitt getrennt. Dabei wird ein Lösungsmittel in den oberen Teil der Kolonne zugeführt, welches die Oxygenate löst. Damit sinkt der Gehalt an Oxygenaten im Vergleich zum Stand der Technik deutlich.

Durch die Wirkung des Lösungsmittels werden die im Kohlenwasserstoffstrom enthaltenden Oxygenate extraktiv entfernt und gelangen in den Sumpf der Kolonne.

Den Kopf der Kolonne verlässt ein oxygenatfreies Produkt. Im Sumpf werden die Oxygenate, das Lösungsmittel und die nicht zum Produkt gehörenden Kohlenwasserstoffe abgezogen. Als oxygenatfreies Produkt wird hierbei ein Produkt bezeichnet, das einen Anteil an sauerstoffhaltigen organischen Verbindungen < 1 ppm aufweist. Die Kolonne wird bei einem Druck von 5 bis 35 bar betrieben.

Als Lösungsmittel können ganz allgemein Mono-Alkohole oder Di-Alkohole verwendet werden. Hierzu eignen sich insbesondere Methanol, Diethylenglykol, Ethanol oder Propanol. Alternativ kann auch N-Methyl-Pyrrolidon (NMP) mit und ohne Wasserzugabe eingesetzt werden.

Als Kohlenwasserstoffe können Olefine (Mono- oder Diolefine), Paraffine, Naphthene oder Aromaten oder eine Mischung dieser Stoffe verwendet werden. Diese Kohlenwasserstoffe können z.B. aus einem katalytischen Reaktionsprozess stammen.

Die sauerstoffhaltigen organischen Verbindungen können als Ether wie z.B. Dimethylether (DME), Ester wie z.B. Methylformiat, Ketone wie z.B. Methyl-Ethyl-Keton oder Aldehyde wie z.B. Formaldehyd, vorliegen. Im einfachen Fall wird das Lösungsmittel, z.B. Methanol (MeOH), im Prozess als Ausgangsstoff der Produktsynthese verwendet, so dass eine direkte Rückführung des Lösungsmittels zusammen mit den gelösten Oxygenaten und Kohlenwasserstoffen in den Prozess möglich ist. Ist dies aus technischen oder wirtschaftlichen Gründen nicht möglich oder ungünstig oder wird anstelle von Alkoholen das Lösungsmittel NMP verwendet, kann das Lösungsmittel durch einen Trennprozess, z.B. Extraktion oder Destination, von den Oxygenaten und Kohlenwasserstoffen getrennt und teilweise oder vollständig in die Extraktivdestillationskolonne zurückgeführt werden.

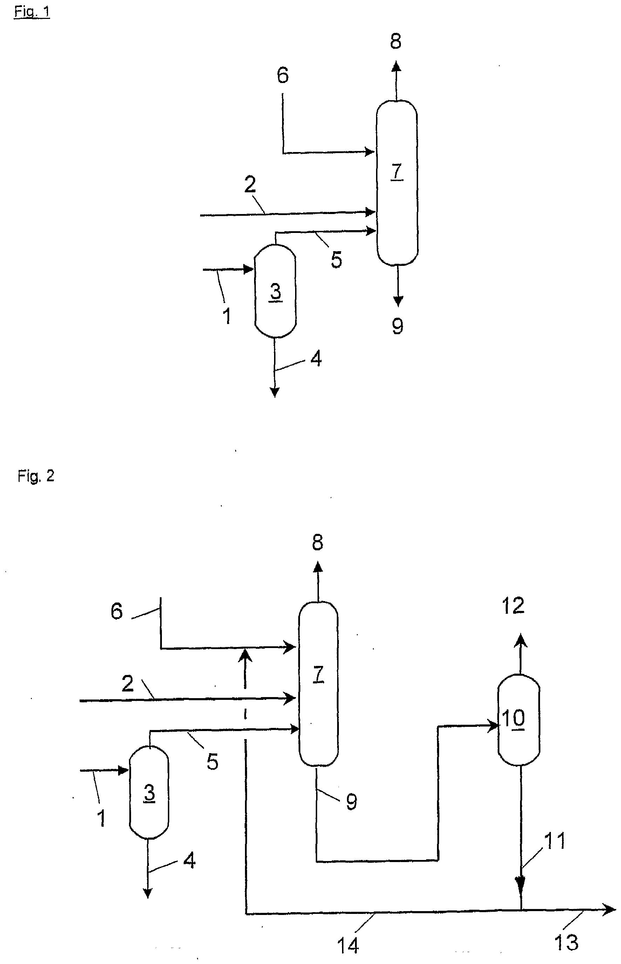

Die Erfindung ist in den Zeichnungen (Fig. 1, Fig. 2) durch ein Verfahrensbeispiel - der Herstellung eines oxygenatfreien C3- Kohlenwasserstoffstroms aus einem Gemisch von C1 - C8 Kohlenwasserstoffen und Oxygenaten, insbesondere DME - dargestellt, das nachstehend erläutert wird.

Eine flüssige Fraktion (1) aus Kohlenwasserstoffen und Oxygenaten wird in eine erste Kolonne (3) aufgegeben. Diese separiert leicht siedende Komponenten C4- und DME (5) von C4+ Kohlenwasserstoffen (4). Die Fraktion mit C4+ Kohlenwasserstoffen (4) wird zu einer nicht dargestellten Destillationskolonne geleitet, in der leichtes Naphtha-Produkt (C4-C6) von C7+ Benzin-Produkt (Paraffine, Olefine, Naphthene und Aromaten) getrennt wird

Die gasförmige Fraktion (2), bestehend aus überwiegend C3- aber auch Restmengen von C4 Kohlenwasserstoffen sowie Oxygenaten, und das C4- Kopfprodukt der ersten Kolonne (5) werden in einer zweiten Kolonne (7) einer Extraktivdestillation zugeführt. Die zweite Kolonne (7) trennt dieses Gemisch bei einem Druck von 21 bis 25 bar in einen leichten und einen schweren Kohlenwasserstoffschnitt. Um zu verhindern, dass Oxygenate als Verunreinigung in das Kopfprodukt gelangen, wird ein flüssiges Lösungsmittel verwendet. Dieses Lösungsmittel wird der Kolonne (7) über Leitung (6) zugeführt. Als Lösungsmittel wird im Beispiel Methanol verwendet. Durch das Lösungsmittel werden die im Kohlenwasserstoffstrom enthaltenden Oxygenate extraktiv entfernt, gelangen in den Sumpf der Kolonne (7) und werden über Leitung (9) abgezogen. Den Kopf der Kolonne (7) verlässt über Leitung (8) ein oxygenatfreies Produkt, das in weiteren nicht dargestellten Destillationskolonnen z.B. zu reinem polymerfähigem Propylen aufgearbeitet werden kann.

Im einfachen Fall (Fig. 1, Beispiel 1) wird angenommen, dass das über Leitung (9) abgezogene Gemisch aus Kohlenwasserstoffen / Oxygenaten / Methanol als Ausgangsmaterial im Grundprozess verwendet werden kann. Es besteht deshalb kein Bedarf, das Lösungsmittel zu regenerieren.

Im Beispiel 2 (Fig. 2) ist eine Prozess-Konfiguration mit einer Regeneration des Lösungsmittels dargestellt. Diese ist dann erforderlich, wenn das Lösungsmittel Methanol im Grundprozess nicht oder nur bedingt eingesetzt werden kann. In diesem Schema wird das über die Leitung (9) abgezogene Gemisch der Kolonne (10) zugeführt. Das Kopfprodukt (12) enthält die leichte Fraktion bestehend aus Kohlenwasserstoffen sowie Oxygenaten, insbesondere DME. Dieses Gemisch (12) kann direkt als Ausgangsstoff in die Produktsynthese des nicht dargestellten Grundprozesses zurückgeführt werden. Das Sumpfprodukt wird über Leitung (11) abgezogen und enthält das regenerierte Lösungsmittel sowie Wasser, welches als Nebenprodukt über die Ströme (1) und (2) eingebracht wird. Der größere Teil dieses Stromes wird über Leitung (14) der Extraktivdestillationskolonne (7) zugeführt. Ein kleiner Teil des Stroms wird über Leitung (13) einer weiteren Aufbereitung z.B. einer Destillationskolonne zugeführt, um Methanol und weitere Oxygenate von Abwasser zu trennen. Der Mengenstrom (13) wird so eingestellt, dass in der Extraktivdestillationskolonne (7) der Wassergehalt in der flüssigen Phase 0,5 - 2 Gew.-%, bevorzugt 1 Gew.-% beträgt und somit das Auftreten einer zweiten flüssigen Phase in der Kolonne verhindert wird.

Im Beispiel 3 (Fig. 2) wird als Lösungsmittel ein Gemisch von NMP (87,5 Gew.-%) und Wasser (12,5 Gew.-%) verwendet. In diesem Fall wird infolge des hohen Siedepunktes des Lösungsmittels im Sumpf der Kolonne (10) der Wassergehalt von 12,5 Gew.-% direkt über die Sumpftemperatur eingestellt, das Lösungsmittel vollständig regeneriert und über (14) der Extraktionsdestillationskolonne (7) zugeleitet. Im Falle einer Verunreinigung des Lösungsmittels -z.B. durch Polymerisation - wird ein kleiner Anteil von (11) diskontinuierlich über (13) aus dem Kreislauf entfernt. In diesem Fall wird eine kleine Menge frisches NMP über Leitung (6) als Ersatz nachgespeist.

### Beispiel 1

Konfiguration ohne Lösungsmittel-Regeneration, mit Lösungsmittel Methanol

| Strom Nummer | 1 | 2 | 4 | 5 | 6 | 8 | 9 |
|---|---|---|---|---|---|---|---|
| Massenstrom kg/hr | | | | | | | |
| MeOH | 408.2 | 98.9 | 0.0 | 408.2 | 30000.0 | 0.1 | 30507.0 |
| DME | 697.2 | 407.1 | 0.0 | 697.1 | 0.0 | 0.1 | 1104.2 |
| H2O | 765.5 | 161.8 | 0.0 | 765.4 | 0.0 | 0.0 | 927.3 |
| C2H4 | 3819.6 | 8908.9 | 0.0 | 3818.6 | 0.0 | 12727.6 | 0.0 |
| C3H6 | 34975.6 | 25857.1 | 30.9 | 34940.8 | 0.0 | 60425.2 | 372.6 |
| 1-C4H8 | 7306.4 | 1440.0 | 3259.7 | 4046.7 | 0.0 | 0.0 | 5486.7 |
| C-C4H8 | 7541.2 | 963.3 | 5477.1 | 2064.2 | 0.0 | 0.0 | 3027.5 |
| T-C4H8 | 7483.5 | 1081.7 | 4876.1 | 2607.4 | 0.0 | 0.0 | 3689.1 |
| I-C4H8 | 7177.3 | 1416.4 | 3337.5 | 3839.6 | 0.0 | 0.0 | 5256.0 |
| C5H10 | 13626.8 | 266.3 | 13615.8 | 11.0 | 0.0 | 0.0 | 277.3 |
| Olefine C6-C8 | 10861.3 | 18.1 | 10861.3 | 0.0 | 0.0 | 0.0 | 18.1 |
| C2H6 | 144.0 | 271.7 | 0.0 | 143.9 | 0.0 | 415.7 | 0.0 |
| C3H8 | 549.3 | 360.4 | 1.6 | 547.6 | 0.0 | 901.0 | 7.0 |
| N-C4H10 | 23478.5 | 4035.9 | 14027.3 | 9449.4 | 0.0 | 0.0 | 13485.2 |
| I-C4H10 | 33454.3 | 7623.1 | 13566.5 | 19886.1 | 0.0 | 0.0 | 27509.3 |
| C5H12 | 55893.0 | 1272.8 | 55827.8 | 65.1 | 0.0 | 0.0 | 1337.9 |
| Paraffine C6 - C8 | 18342.2 | 27.7 | 18342.2 | 0.0 | 0.0 | 0.0 | 27.7 |
| Alkohole C2 - C5 | 297.8 | 0.1 | 297.8 | 0.0 | 0.0 | 0.0 | 0.1 |
| Naphthene C5 - C6 | 6203.3 | 4.1 | 6203.3 | 0.0 | 0.0 | 0.0 | 4.1 |
| Aromaten | 4277.7 | 0.3 | 4277.7 | 0.0 | 0.0 | 0.0 | 0.3 |
| Ketone C3 - C5 | 396.1 | 1.4 | 396.1 | 0.0 | 0.0 | 0.0 | 1.4 |
| Aldehyde C1 - C3 | 266.3 | 30.7 | 184.4 | 81.9 | 0.0 | 0.0 | 112.6 |
| Methylformiat | 94.9 | 2.6 | 94.8 | 0.0 | 0.0 | 0.0 | 2.7 |
| H2 + CH4 | 505.0 | 3552.5 | 0.0 | 503.6 | 0.0 | 4056.2 | 0.0 |
| Massenstrom kg/hr | 238565.0 | 57803.0 | 154678.0 | 83876.8 | 30000.0 | 78525.7 | 93154.2 |
| Temperatur °C | 100.0 | 77.4 | 161.4 | 44.6 | 20.0 | 36.8 | 103.1 |
| Druck bar | 24.6 | 22.0 | 22.0 | 21.7 | 10.0 | 21.5 | 22.0 |
| Dichte kg/m³ | 495.0 | 36.0 | 439.7 | 505.4 | 790.8 | 39.8 | 534.5 |
| Molmasse kg/kmol | 59.4 | 39.6 | 70.0 | 46.4 | 32.0 | 36.9 | 44.8 |

### Beispiel 2

Konfiguration mit Lösungsmittel-Regeneration und -Kreislauf, mit Lösungsmittel Methanol

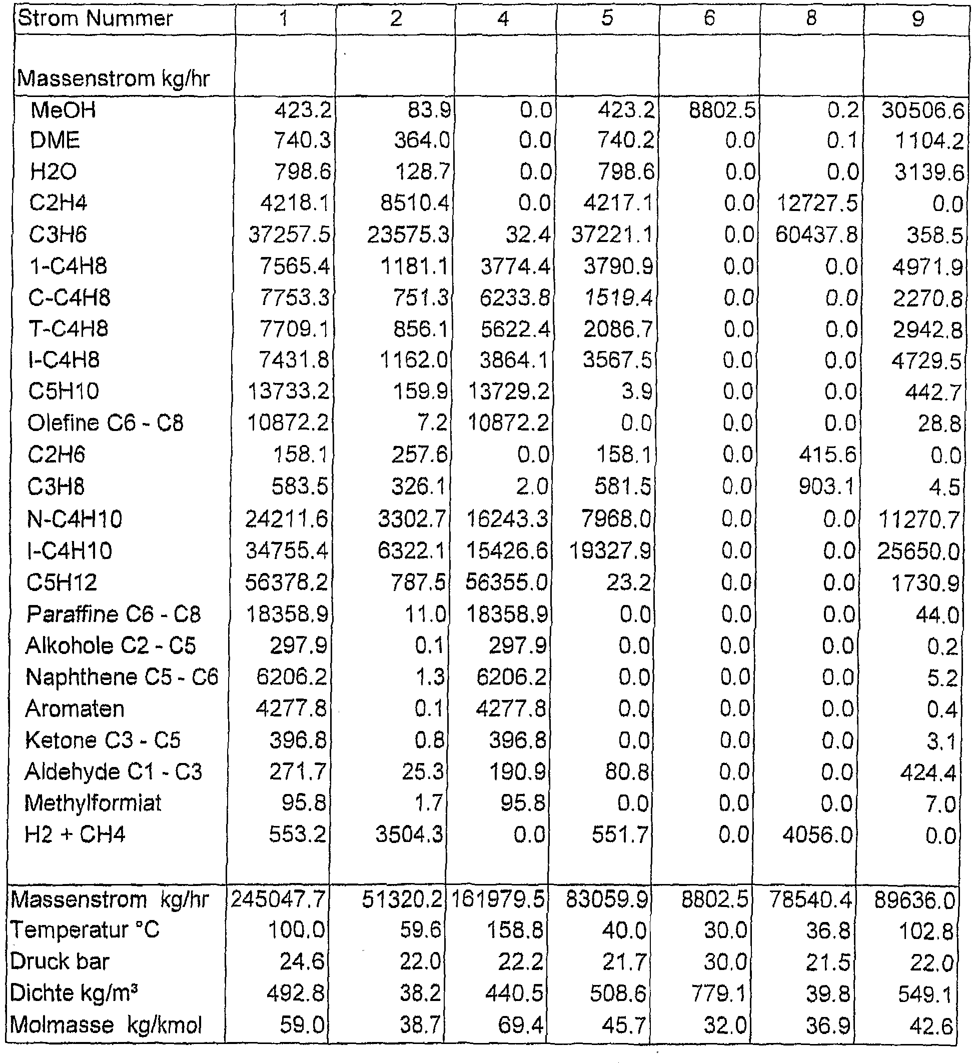

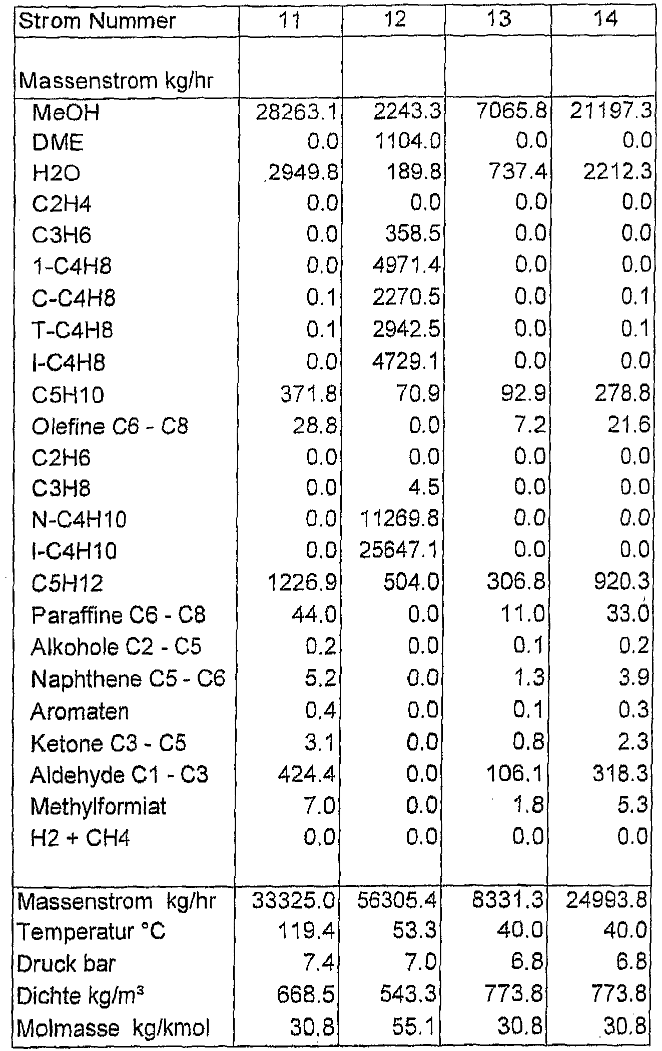

### Beispiel 3

Konfiguration mit Lösungsmittel-Regeneration und -Kreislauf, mit Lösungsmittel NMP/H2O

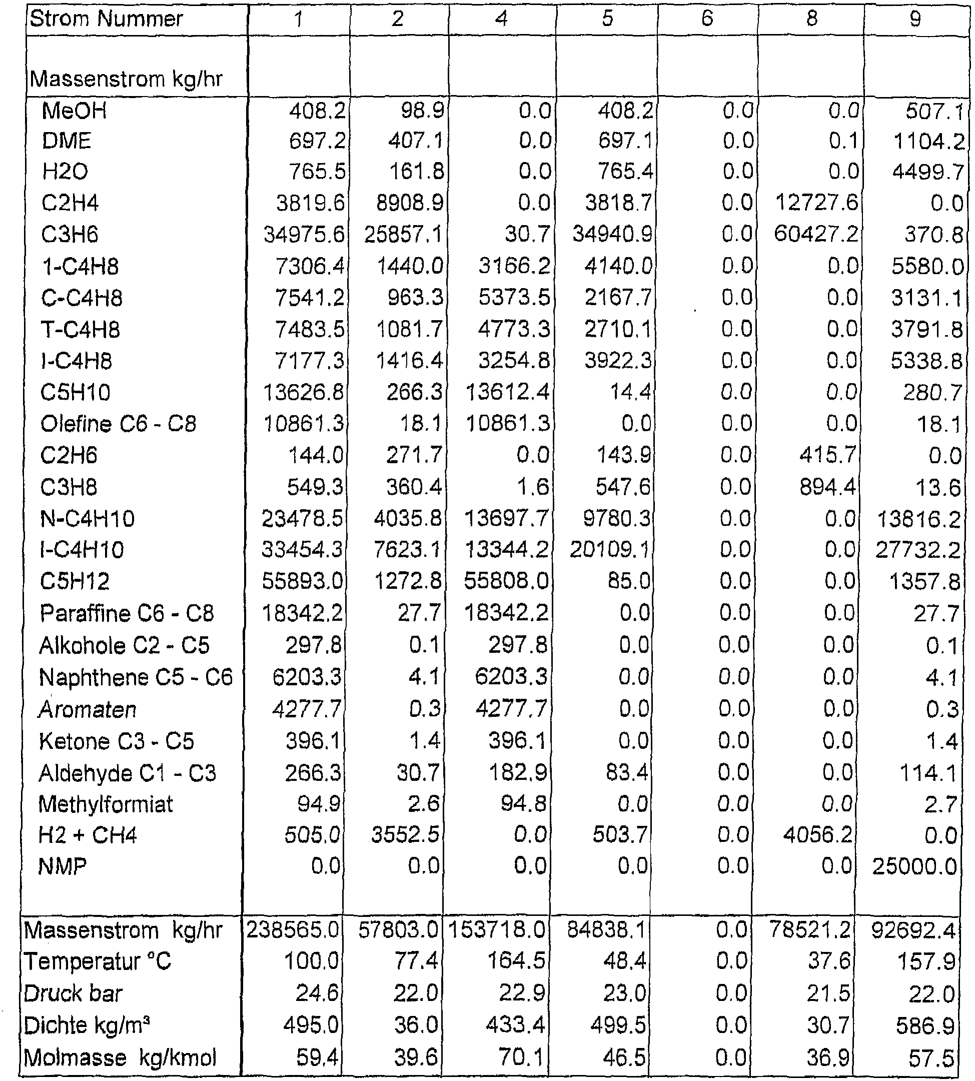

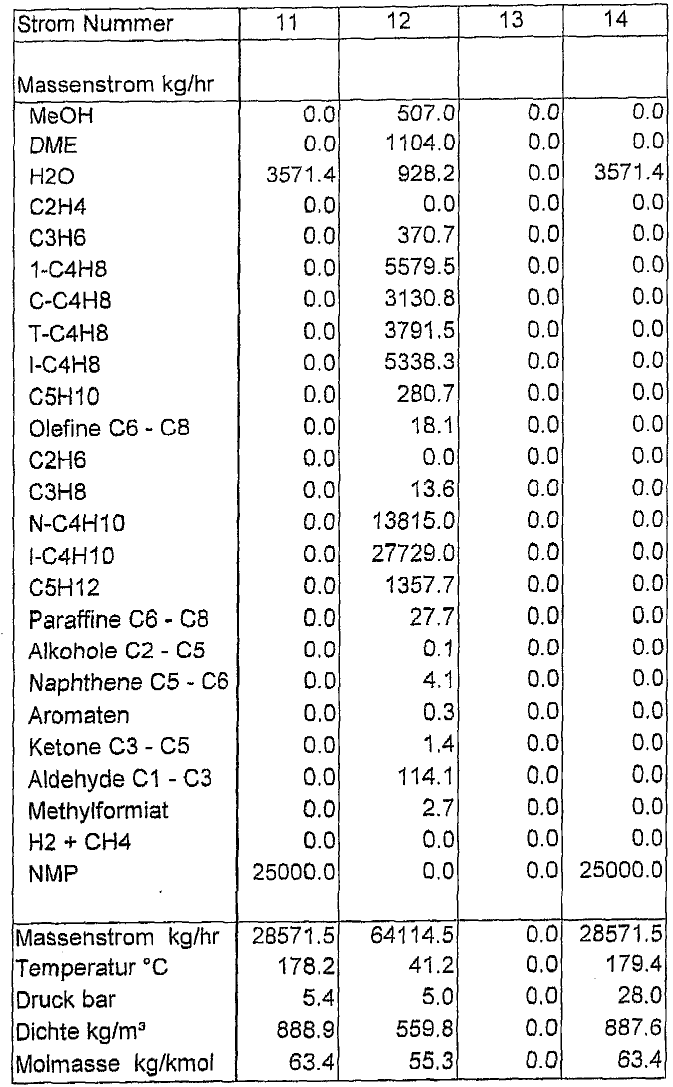

## Patentansprüche

1. Verfahren zum Entfernen von sauerstoffhaltigen organischen Verbindungen, Oxygenaten, aus Gemischen verschiedener Kohlenwasserstoff-Verbindungen, **dadurch gekennzeichnet, dass** eine Kohlenwasserstoffe und Oxygenate enthaltende Flüssigphase (1) einer ersten Kolonne (3) aufgegeben wird, wobei in dieser ersten Kolonne (3) destillativ eine leichte Fraktion als Kopfprodukt (5) abgetrennt wird, und dass eine schwerere C₄₊-Fraktion aus dem Boden der Kolonne (3) entfernt wird, und dass die leichte Fraktion (5) und räumlich getrennt davon ein gasförmiges Gemisch (2), umfassend C₃-Kohlenwasserstoffe, C₄-Kohlenwasserstoffe und Oxygenate, einer zweiten Kolonne (7) aufgegeben werden, wobei die Aufgabestelle des gasförmigen Gemisches (2) oberhalb der Aufgabestelle der leichten Fraktion (5) liegt, wobei in der Kolonne (7) eine destillative Trennung in eine leichte und eine schwere Kohlenwasserstofffraktion erfolgt, wobei ein zusätzliches Lösungsmittel (6) in den oberen Teil der Kolonne (7) zugeführt wird, welches die Oxygenate löst und im Sumpfprodukt (9) der Kolonne ausschleust, so dass den Kopf der Kolonne (7) ein oxygenatfreies Kohlenwasserstoff-Produkt (8) verlässt und aus dem Boden der Kolonne (7) ein Gemisch aus Oxygenaten, Lösungsmittel und restliche Kohlenwasserstoffe (9) abgezogen wird.

2. Verfahren nach Anspruch 1, **dadurch gekennzeichnet, dass** als Lösungsmittel Mono-Alkohole oder Di-Alkohole verwendet werden.

3. Verfahren nach Anspruch 1, **dadurch gekennzeichnet, dass** als Lösungsmittel Methanol, Diethylenglykol, Ethanol oder Propanol verwendet werden

4. Verfahren nach Anspruch 1, **dadurch gekennzeichnet, dass** als Lösungsmittel N-Methyl-Pyrrolidon (NMP) verwendet wird.

5. Verfahren nach Anspruch 1 bis 4, **dadurch gekennzeichnet, dass** die zweite Kolonne (7) bei einem Druck von 5 bis 35 bar betrieben wird.

6. Verfahren nach Anspruch 1 bis 5, **dadurch gekennzeichnet, dass** die Gas- und Flüssigkeitsphase (2) und (1) aus einem katalytischen Reaktionsprozess stammen.

7. Verfahren nach Anspruch 1 bis 6, **dadurch gekennzeichnet, dass** das Gemisch aus Oxygenaten, Lösungsmitteln und restlichen Kohlenwasserstoffen (9) aus der zweiten Kolonne (7) abgezogen und einer dritten Kolonne (10) zugeführt wird, wobei aus dem Kopf der Kolonne (10) eine leichte Fraktion aus Kohlenwasserstoffen, Oxygenaten und DME (12) abgezogen wird, und aus dem Boden der Kolonne (10) regeneriertes Lösungsmittel und Wasser (11) entfernt und teilweise über Leitung (6) der zweiten Kolonne (7) wieder zugeführt wird.

8. Verfahren nach Anspruch 7, **dadurch gekennzeichnet, dass** als Lösungsmittel ein Gemisch von NMP mit Wasser verwendet wird.

9. Verfahren nach Anspruch 8, **dadurch gekennzeichnet, dass** der Wasseranteil 5 - 20 Gew.-% beträgt.

10. Verfahren nach Anspruch 9, **dadurch gekennzeichnet, dass** in der Kolonne (7) der Wassergehalt in der flüssigen Phase 0,5 - 2 Gew.-%, bevorzugt 1 Gew.-% beträgt.

## Claims

1. Process for removing oxygen-containing organic compounds (oxygenates) from mixtures of different hydrocarbon compounds, **characterized in that** a liquid phase (1) containing hydrocarbons and oxygenates is added to a first column (3), wherein in this first column (3) a light fraction is separated by distillation as head product (5), and a heavier C4+ fraction is removed from the bottom of the column (3), and **in that** the light fraction (5) and a gaseous mixture of hydrocarbons and oxygenates (2) are added to a second column (7), wherein a distillative separation into a light and a heavy hydrocarbon fraction takes place, wherein an additional solvent (6) is fed into the upper part of the column (7), which dissolves the oxygenates and discharges them in the bottom product (9) of the column, so that an oxygenate-free hydrocarbon product (8) leaves the head of the column (7) and a mixture of oxygenates, solvents and residual hydrocarbons (9) is drawn off from the bottom of the column (7).

2. Process according to claim 1, **characterized in that** mono-alcohols or dialcohols are used as the solvent.

3. Process according to claim 1, **characterized in that** methanol, diethylene glycol, ethanol or propanol are used as the solvent.

4. Process according to claim 1, **characterized in that** NMP (N-methylpyrrolidone) is used as the solvent.

5. Process according to claim 1 to 4, **characterized in that** the second column (7) is operated at a pressure of 5 to 35 bar.

6. Process according to claim 1 to 5, **characterized in that** the gas and liquid phases (2) and (1) originate from a catalytic reaction process.

7. Process according to claim 1 to 6, **characterized in that** the mixture of oxygenates, solvents and residual hydrocarbons (9) is withdrawn from the second column (7) and fed to a third column (10), a light fraction of hydrocarbons, oxygenates and DME (12) is withdrawn from the head of the column (10), and regenerated solvent and water (11) are withdrawn from the bottom of the column (10) and partially fed back to the second column (7) via a line (6).

8. Process according to claim 7, **characterized in that** a mixture of NMP with water is used as the solvent.

9. Process according to claim 8, **characterized in that** the water content is 5 - 20 wt.-%

10. Process according to claim 9, **characterized in that** in the colon (7) the water content in the liquid phase is 0.5 - 2% by weight, preferably 1% by weight.

## Revendications

1. Procédé pour éliminer des composés organiques contenant de l'oxygène (produits oxygénés) à partir de mélanges de différents composés hydrocarbonés, **caractérisé en ce qu'**une phase liquide (1) contenant des hydrocarbures et des produits oxygénés est appliquée sur une première colonne (3), une fraction légère étant séparée par distillation dans cette première colonne (3) en tant que produit de tête (5), et **en ce qu'**une fraction plus lourde en C₄₊ est éliminée du fond de la colonne (3) et **en ce que** la fraction légère (5) et, de manière spatialement séparée de celle-ci, un mélange gazeux (2), comprenant des hydrocarbures en C₃, des hydrocarbures en C₄ et des produits oxygénés, sont appliqués sur une deuxième colonne (7), le site d'application du mélange gazeux (2) se situant au-dessus du site d'application de la fraction légère (5), une séparation par distillation en une fraction hydrocarbonée légère et en une fraction hydrocarbonée lourde ayant lieu dans la colonne (7), un solvant (6) supplémentaire étant introduit dans la partie supérieure de la colonne (7), qui dissout les produits oxygénés et les évacue dans le produit du fond (9) de la colonne de manière telle qu'un produit hydrocarboné (8) exempt de produits oxygénés quitte la tête de la colonne (7) et qu'un mélange de produits oxygénés, de solvant et d'hydrocarbures résiduels (9) est soutiré du fond de la colonne (7).

2. Procédé selon la revendication 1, **caractérisé en ce qu'**on utilise, comme solvant, des monoalcools ou des dialcools.

3. Procédé selon la revendication 1, **caractérisé en ce qu'**on utilise, comme solvant, du méthanol, du diéthylèneglycol, de l'éthanol ou du propanol

4. Procédé selon la revendication 1, **caractérisé en ce qu'**on utilise, comme solvant, de la NMP (N-méthyl-pyrrolidone).

5. Procédé selon la revendication 1 à 4, **caractérisé en ce que** la deuxième colonne (7) est exploitée à une pression de 5 à 35 bars.

6. Procédé selon la revendication 1 à 5, **caractérisé en ce que** la phase gazeuse (2) et la phase liquide (1) proviennent d'un procédé de réaction catalytique.

7. Procédé selon la revendication 1 à 6, **caractérisé en ce que** le mélange de produits oxygénés, de solvants et d'hydrocarbures résiduels (9) est soutiré de la deuxième colonne (7) et alimenté dans une troisième colonne (10), une fraction légère d'hydrocarbures, de produits oxygénés et de DME (12) étant soutirée de la tête de la colonne (10) et du solvant régénéré et de l'eau (11) étant éliminés du fond de la colonne (10) et recyclés partiellement via la conduite (6) dans la deuxième colonne (7).

8. Procédé selon la revendication 7, **caractérisé en ce qu'**on utilise, comme solvant, un mélange de NMP et d'eau.

9. Procédé selon la revendication 8, **caractérisé en ce que** la proportion d'eau est de 5-20% en poids.

10. Procédé selon la revendication 9, **caractérisé en ce que**, dans la colonne (7), la teneur en eau dans la phase liquide est de 0,5-2% en poids, de préférence de 1% en poids.
